# EUROPEAN PATENT SPECIFICATION

(11) **EP 3 544 734 B1**
(45) Date of publication and mention of the grant of the patent: **07.07.2021**
(21) Application number: 17873396.0
(22) Date of filing: 21.11.2017
(51) Int. Cl.: B01L 3/00, G01N 33/543, G01F 11/10, G01N 21/03, G01F 11/12

(54) **LAB-ON-A-CHIP DEVICE FOR PERFORMING ANALYSES**
LABOR-AUF-CHIP-VORRICHTUNG ZUR DURCHFÜHRUNG VON ANALYSEN
DISPOSITIF DE LABORATOIRE SUR PUCE POUR EFFECTUER DES ANALYSES

(30) Priority: 28.11.2016 FI 20165903
(43) Date of publication of application: 02.10.2019
(73) Proprietor: Labmaster Oy, 20200 Turku (FI)
(72) Inventor: PERÄLÄ, Petri, 21530 Paimio (FI); KORPELA, Timo, 20610 Turku (FI); WAHLROOS, Tony, Turku 20900 (FI); KORPELA, Saara, 20500 Turku (FI)
(74) Representative: Berggren Oy
(86) International application number: PCT/FI2017/050797
(87) International publication number: WO 2018/096206

(56) References cited:
- WO-A1-2005/068967
- WO-A1-2005/068967
- WO-A1-2011/088582
- WO-A1-2011/088582
- WO-A1-2014/122490
- JP-A- 2008 157 723
- JP-A- 2008 157 723
- US-A- 4 726 237
- US-B2- 8 646 316
- US-B2- 9 463 457
- US-B2- 9 463 457

## Description

### FIELD OF INVENTION

This disclosure relates to methods of chemical and biochemical analyses, especially when used in non-conventional conditions outside professional laboratory facilities. In particular, it is disclosed an easy-to-manufacture single-used cartridge which can carry out sophisticated processes required to perform common steps especially required in clinical analyses.

### BACKGROUND

Chemical and biochemical analyses of specific molecules of interest are an essential part of everyday work in research laboratories, in industrial companies, hospitals, research institutes, and even at homes. There is an increasing need to conduct analyses in a place of the need of the results or for providing convenient situations for the analyses. This is exemplified by clinical analyses of patient near-bed in hospitals, in ambulances, environmental samples in nature, or in industry near the process, so that counter measures can be taken immediately. A common approach to this direction is so-called "lab-on-a-chip" or "lab-on-chip" technique (later on abbreviated as LOC technique) which means an easy-to-use device including necessary means and reagents to perform the needed analyses from applying a sample to getting the results.

Typically, a chemical or biochemical analysis involves first a sampling step, which aims to provide a representative and quantitative sample. Then the analysis contains a sample preparation step which has the purpose of purifying the sample, or excluding components which can interfere in the analysis. This step may include precipitation, de-proteinization, dilution, centrifugation, dialysis, filtration, or other related procedures. The next step is bringing the sample into contact with specific reagents enabling quantification and/or qualification of the substance of interest (analyte) in the sample. After a reaction in predetermined conditions, qualitative or quantitative results are obtained either by visual inspection or most often by using a physical method such as measurement of colour intensity or fluorescence.

Various LOC methods are especially suitable to perform analyses near the place of need, *in situ,* or point-of-care, all of which will be abbreviated hereafter collectively as POC.

The commonly used logic has been that, because multiple operations must be conducted on a single LOC-device, such a device performing the analysis must be small. This has led to sophisticated LOC-chips containing micrometre -wide channels, pumps, valves, actuators, etc. This very important approach of developing micro-technical devices, as such, has unrecognized drawbacks of LOC devices for POC applications. The work with micro-dimensions of active components demands considering novel physical and chemical principles against utmost challenges in manufacturing technologies, purity of materials, high costs of the manufacturing machines in working ultrapure facilities. Material savings can hardly compensate those costs. Moreover, if the applied micro-technology is not of the highest quality, the reproducibility of the, mostly single-use tests will be poor. These pursues for miniaturization are, therefore, controversial to real needs, since in most applications LOC devices cannot be microscopically small since they must be handled by human beings. A tiny disposable LOC device may conceivably be placed on a multi-use handheld platform but again a severe drawback is the need for manipulating tiny things. Moreover, such a platform can be contaminated by microbes, or cause carry over and other harms to the analysis system. Hence, in spite of the seeming advantages, the micro-technological devices cannot be applied into practise since the small size and material savings cannot be effectively exploited. Meanwhile the micro-dimensions create technical problems that are difficult to solve and aggravate the potentially poor performance of the device.

US 9,463,457 B2 (Conventa Ltd.) provides a single-use cartridge for measuring analytes. A cartridge involving two moving parts, i.e. a body and one moving part is described. These two parts can move in respect to each other resulting in a flow of liquids inside the cassette. However, this device does not solve the problem of accurate dosing of the sample and its purification and therefore it does not fulfil the requirements of the LOC devices.

### SUMMARY OF THE INVENTION

This invention solves the above described problems and more.

It is an object to provide a versatile, single use, accurate LOC device.

An object of the invention is to provide a lab on a chip device to accurately measure an exact volume of a sample and conduct a chemical or biochemical assay, said device comprising : a chassis and a liquid reservoir, a sample preparation unit (SPU) and a piston comprising a sample cavity comprising reagents , wherein the SPU and the piston slide in relation to each other and inside the chassis, and the sliding movement opens and closes sample openings on the SPU thereby enabling one or more of the following: measuring an exact predetermined volume of a sample to the sample cavity, adding a standardized amount of one or more reagents into the sample cavity, filtering a sample before entering the sample cavity, and closing the device, and wherein the sample cavity can be moved by sliding of the piston into the liquid reservoir for further reactions or washing.

It is an object to provide a lab on a chip device for chemical or biochemical assays, said device comprising a hollow chassis having a first open end, a second open end and a sample application hole; a detachable liquid reservoir having a bottom, at least partially transparent closed wall and an upper open end, said reservoir containing reagents necessary for the assay, and said reservoir being connectable from the upper end to the first open end of the chassis; a transport unit having a longitudinal axis and moving into the chassis from its second open end, said transport unit comprising a piston frame with a distal end and a proximal end with an end plate and having a sample cavity containing predetermined reagents and optionally covered with a filtering unit; anda sample preparation unit (SPU) on top of the transport unit parallel to the longitudinal axis and being capable of sliding along the longitudinal axis, said SPU having one or more of the functions selected from, filtration, standardization of sample volume, addition of a standardized amount of a reagent, and closing the device; said SPU comprising: an upper slide having a sample application opening and an air hole both extending through the slide; anda lower slide having a sample measuring opening, said opening extending through the slide and having a predetermined exact volume; the device having a sample application position, a sample standardization position, a sample transfer position, and a sample washing position, wherein in the sample application position the upper slide and the lower slide of the SPU are positioned inside the chassis such that the sample application hole of the chassis, the sample application opening of the upper slide and the sample measuring cavity of the lower slide are aligned with each other, thus allowing a sample to enter through the sample application hole and fill the application opening and the sample measuring opening; in the sample standardization position the upper slide being positioned such that the sample application opening of the upper slide and the sample measuring opening of the lower slide are misaligned, and contents of the sample application opening are thus contained in the opening while the sample measuring opening contains the exact predetermined volume of the sample; in sample transfer position the upper slide and the lower slide of the SPU and the transport unit are positioned inside the chassis such that the sample application hole of the chassis, the air hole of the upper slide, the sample measuring opening of the lower slide and the sample cavity in the transport unit are aligned with each other, thereby allowing the exact predetermined volume in the measuring opening to enter the sample cavity and allowing an option to add subsequent reagents to the sample cavity through the aligned openings; in the sample washing position the SPU is positioned completely inside the chassis and the sample transfer unit is positioned such that the sample cavity locates in the liquid reservoir and the end plate of the piston frame tightly closes the device.

It is a further object to provide a lab on a chip device for chemical or biochemical assays, said device comprising: a hollow chassis having a first open end, a second open end and a sample application hole; a detachable liquid reservoir having a bottom, at least partially transparent closed wall and an upper open end optionally covered with a membrane, said reservoir containing reagents necessary for the assay, and said reservoir being connectable from the upper end to the first open end of the chassis; a transport unit being a piston having a longitudinal axis and moving into the chassis from its second open end, said transport unit having a piston frame and a sample cavity optionally covered by a filtering unit and containing predetermined reagents; and a sample preparation unit (SPU) locating on top of the transport unit and being capable of sliding along the longitudinal axis of the piston and comprising a slide having a sample application opening extending through the slide, and the SPU having one or more of the functions selected from, filtration, standardization of sample volume, addition of a standardized amount of a reagent, and closing the device; said device having a sample application position, a reaction position, and a washing position, wherein in the sample application position the SPU and the transport unit being positioned inside the chassis such that the sample application hole of the chassis, the sample application opening in the slide, and the sample cavity in the transport unit are aligned, thus allowing a sample to enter through the sample application hole and sample application opening into the sample cavity; in the reaction position the slide being positioned such that the sample hole of the chassis and the sample opening of the slide are misaligned and the sample application opening of the slide thus being closed; in the washing position the transport unit being positioned such that the sample cavity locates in the liquid reservoir allowing the reagent in the reservoir wash the sample cavity.

A kit for performing chemical and biochemical analyses comprises: one or more hollow chassis units, each having a first open end, a second open end and a sample application hole; one or more sample preparation units, each having at least one hole with an exact volume for a sample;one or more transport units each having a piston frame with a sample cavity and each having same or different reagents in the sample cavity; and one or more liquid reservoirs having same or different contents, wherein any of the liquid reservoirs are attachable to any of the first open ends of the chassis units,any of the sample preparation units are capable of sliding on top of any of the piston frames, and any combination of the piston frames and sample preparation units fits through the second open end of the chassis.

Still another object is to provide a method to accurately measure an exact volume of a sample and conduct a chemical or biochemical assay, said method comprising the steps of: providing a lab-on-a-chip -device having a chassis, a sample preparation unit and a piston comprising a sample cavity, wherein the sample preparation unit (SPU) and the piston slide in relation to each other and inside the chassis, and the sliding movement opens and closes sample openings on the SPU thereby enabling one or more of the following: measuring an exact predetermined volume of a sample, adding a standardized amount of one or more reagents, filtering a sample, and closing the device.

The problem was set here different from the customary thinking: how to make LOC devices which avoid the drawbacks of micro-technology while being able to employ the modern standard manufacturing methods. The problem was solved with macro-scale mechanical components. The device includes solid and liquid reagents, place for wastes, sample standardization and treatment unit, incubation unit, and place for recording the results. The positioning of the sample and movement of the liquids was obtained by three or more logically sliding components in the device which can be constructed to form a closing cassette.

The present disclosure describes significantly improved method and construction of LOC devices which overcome the existing drawbacks of sampling and sample treatment with additional sliding units. This disclosure illustrates how mechanical movements can be organized to affect complicated analysis steps which allow anyone to make demanding chemical analyses.

### BRIEF DESCRIPTION OF THE DRAWINGS

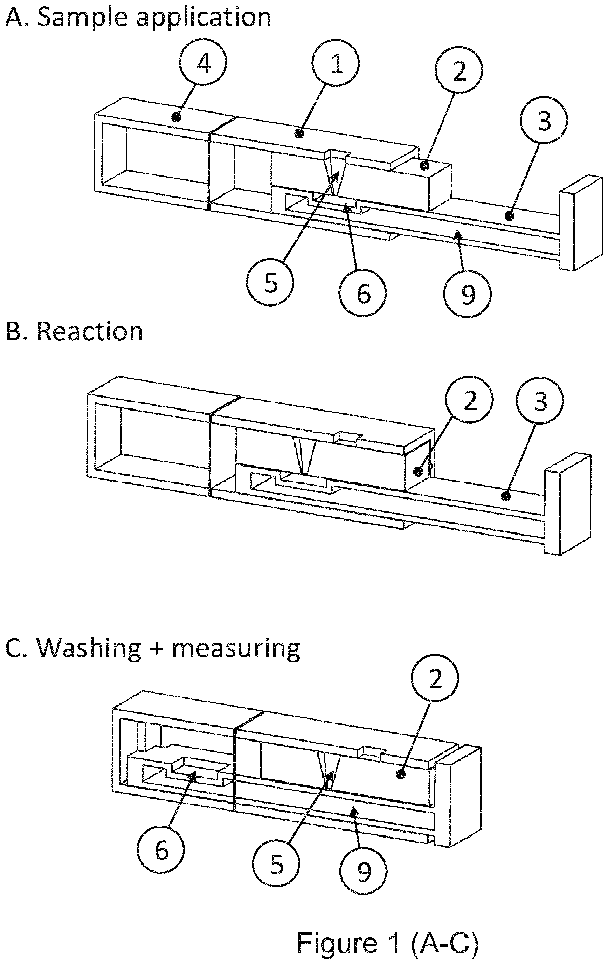

Figure 1 depicts an axial cross-sectional view of overall construction of the LOC device according to the present disclosure and illustrates the steps of the function (A-C). The device includes at least three separate parts, operations of which are logically connected to each other. The cover part (chassis) (1) is connectable to liquid reservoir (4) which together forms the outer part of the device or cassette (elements 1 and 4 together). The moving part inside the cover comprises a sample transport unit or piston (3) and sample preparation unit or SPU (2) which SPU can slide over the piston along the direction of the longitudinal axis inside the chassis. The distal end or head part of the piston (3) contains sharp elements such as edges, spikes or knives which break the seal or membrane covering opening of the liquid reservoir (4). The sample transport unit or piston comprises a sample cavity (6) which along with the sample preparation unit contains most of the chemical and biochemical reagents needed to carry out the most steps of the analyses. The waste liquids are introduced into a waste container (9) through appropriate holes (not shown) near the sample cavity (A, C; 6). SPU (2) may contain several functions and thereby moves logically above the piston part (3). In step A, sample is introduced through a sample application hole in the chassis aligned with the sample application opening (5) in the SPU. SPU is in such a position that the sample application opening (5) in SPU (2) is aligned with the sample cavity (6) and thus the sample enters the opening of (5) in SPU. In step B the hole in the chassis is closed when sample preparation unit (2) is moved (B). Fig. 1 (C) illustrates the final stages of washing step before final closing of the cassette followed by the measurement of the amount of the analyte.

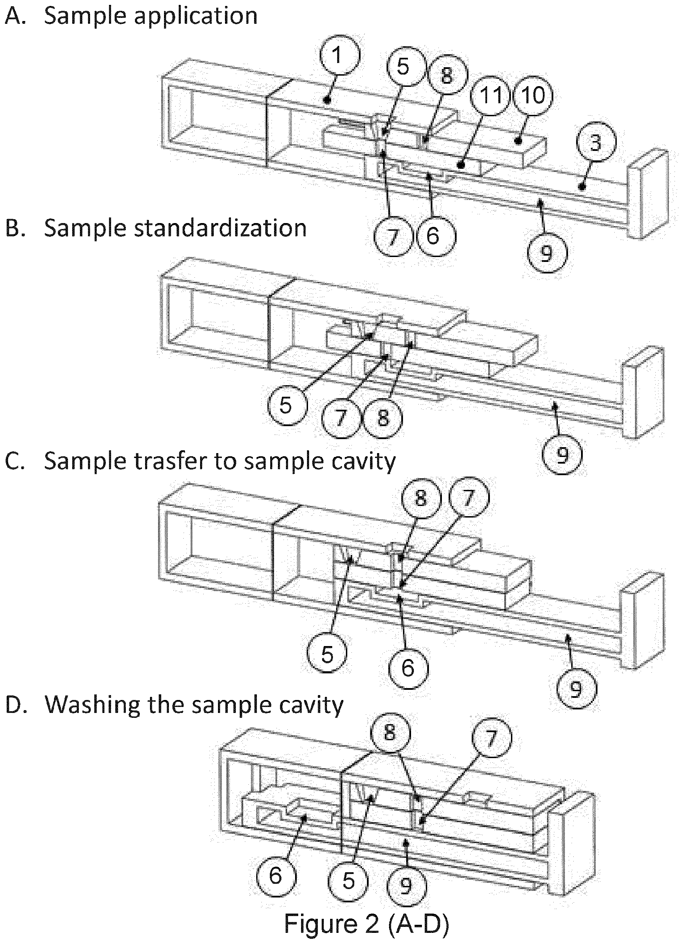

Figure 2 (A-D) illustrates an axial cross-sectional view in more details in another more complicated variant of the sample preparation unit (SPU; element 2 in Fig. 1) with the option of a quantitative sampling unit. SPU is now composed of two sliding plates (an upper and a lower slide, 10 and 11, respectively). In the sample application position (A) where the sample hole in the chassis 1, as well as the sample opening 5 in the upper slide and the measuring opening 7 in the lower slide are aligned, a sample is applied in an excess to the final need of the volume. The sample fills the measuring opening (7), the bottom of which is closed by the piston frame. In sample standardization position B, the applied sample is divided into two parts, the lower one being exactly known in volume and locating in the sample measuring opening 7 in the lower slide 11 while excess of the sample locating in the sample opening 5 in the upper slide 10 is transferred to an idle or waste position such that the sample opening (5) is no more aligned with the measuring opening 7. In sample transfer position C, the upper slide 10 is moved to sample transfer position where an air hole 8 in the upper slide 10 is aligned with the sample hole in the chassis and with the measuring opening 7 in the upper slide 10 as well as with the sample cavity 6 in the transport unit 3, allowing a quantitative volume (determined by volume of 7) of the sample to drop into the sample cavity 6 which sample cavity may be covered for example with a filter (not shown) through which the sample would penetrate. The air hole 8 can also conveniently serve as a reservoir of a liquid for dilution of the sample, or contain other reagents for adding to the sample. In sample washing position D the transport unit 3 has been pushed through the membrane closing the liquid reservoir entrance and the sample cavity 6 is in the liquid reservoir near the bottom of the reservoir and the measuring can take place through a window or a transparent wall of the liquid reservoir (4 in Fig. 1).

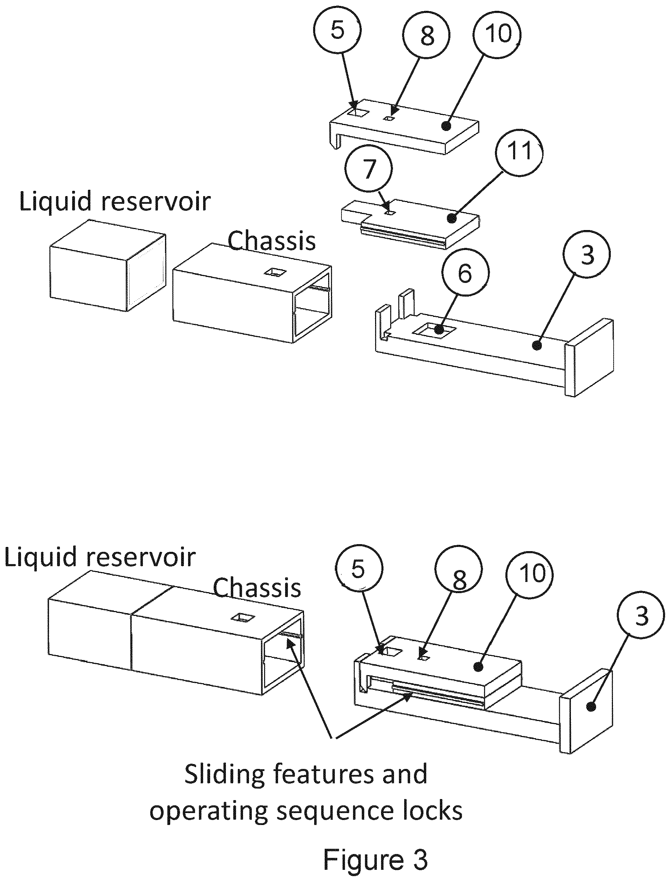

Figure 3 illustrates schematically another construction a LOC device where the lower slide 11 and the upper slide 10 form an assembly fitted to move together to accomplish the sampling. The transport unit 3 serves also here as the displacement pump of liquid from the liquid reservoir waste reservoir inside 3. Sliding feature and operating sequence locks of 2 are indicated by a black line on the outside side of 2 and inside of the chassis.

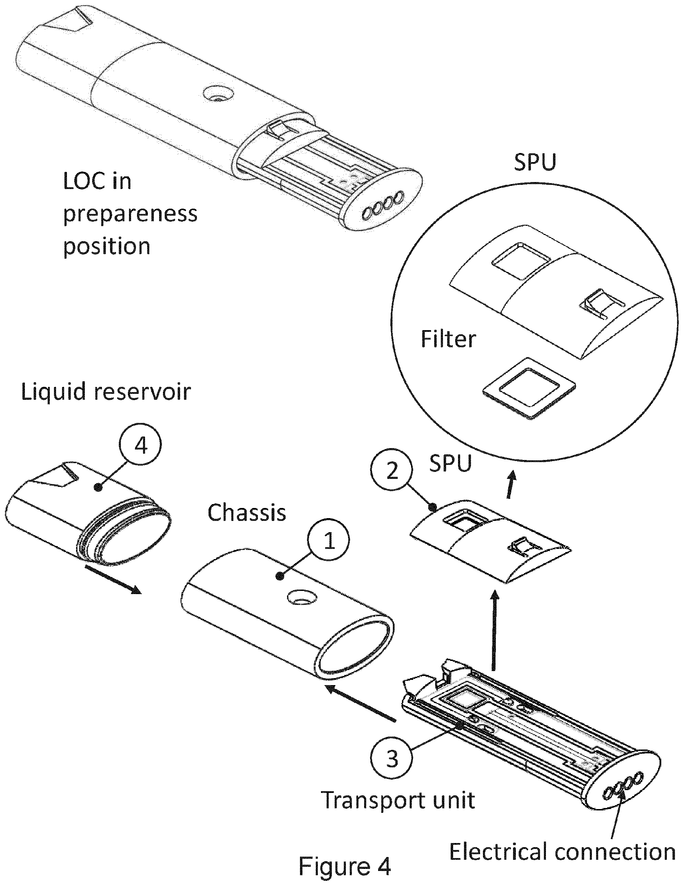

Figure 4 illustrates a construction for electro-chemiluminesence or, e.g. for amperometric analyses. It carries electrical connections on the transport unit or piston 3. Electrical contact to the measuring instrument is shown from the end of the transport unit. SPU 2 contains here slides 10 and 11 and a filter (insert, a view from end of SPU). Liquid reservoir 4 is made as a separate unit from chassis 1. The whole cassette in preparedness position is depicted, as well.

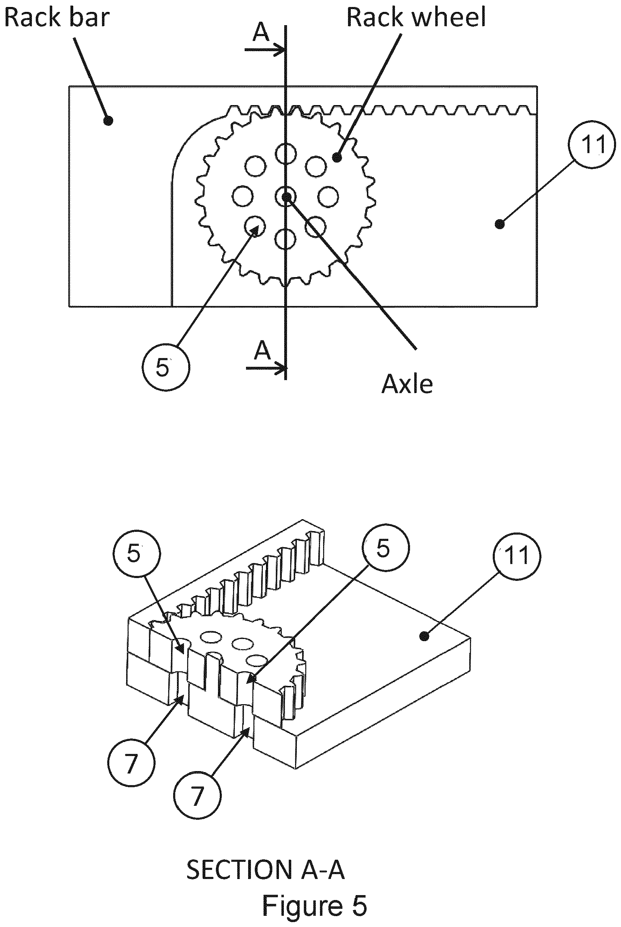

Figure 5 (view from side and top) illustrates yet another construction of SPU which is formed of a slide 11 and a rotating rack wheel or cog located on it. The rotating wheel is actuated with a toothed bar which represents the upper slide 10 in Fig. (2). End view: the rotating wheel is shafted to the lower slide 11. For numbers 11, 5 and 7, see Fig. 2. This SPU can be conveniently used to apply two samples into one cassette. View from the top: rack wheel contains sampling and optionally reagent holes. The rack bar actuates the exact position of the rack wheel which represents the related function as the slide 10 in Fig. 2 for dosing the sample volume.

### DETAILED DESCRIPTION OF THE INVENTION

The "Lab-on-a-chip" (later herein abbreviated as LOC) is a concept allowing a chemical analysis to be conducted with a device which contains all needed functions to perform the analysis. A lab-on-a-chip (LOC) is a device that integrates one or several laboratory functions on a single chip of only from a few square mm to square cm to achieve automation and high-throughput analytical screening. LOCs may deal with the handling of extremely small fluid volumes down to less than microliters. A common trend in making LOC devices is to apply various micro-technological components. As described above, the micro-components and -channels may not be reasonable since the LOC device shall be easily handled by non-professionals. The micro- and nanotechnological manufacturing processes bring about extra challenges. The analyses made with LOC devices are in majority of cases relatively simple and the gained advantages of the smallness are unpractical and mainly of academic interest only. This leads to a conclusion that micro-technological LOC devices are not optimal for solving the practical analytical problems and therefore other approaches were searched for. The question to be answered was, therefore, how to make hand-held LOC devices which can be manufactured with standard methods but which can carry out required analyses in more convenient way than the currently known art.

The present disclosure exploits commonly available printing and injection molding techniques and fulfils the posed problem with minimal components. The present disclosure suits especially well to medical and biochemical *in vitro* diagnostic analyses where the high specificity is acquired by the bioaffinity reactions while the performance is of the highest quality. The described invention can be applied with small modifications for simpler chemical and biochemical analyses, as well as to analyses which presently are carried out by different means. Such simple nonconventional LOC applications are exemplified here with microbial cultivation tests.

### Definitions

*LOC* is abbreviation for the term Lab-on-a-Chip or Lab-on-Chip.

*Cassette* refers in the present disclosure to a LOC device which can be closed and will not leak liquids or does not create contaminations.

*Preparedness stage* of the cassette refers to cassette before a sample is applied to it.

*POC* refers to point-of-care, *in situ* or near-bed tests.

*Micro-technology* refers here to technology of dimensions of 1-100 micro-meters.

*Nanotechnology* refers here to dimensions of 1-100 nano-meters.

*Macroscopic* refers here to dimensions of LOC device in the construction that are at least 10-fold larger than in micro-technological systems. Macroscopic component refers to component of the largest dimension more than 0.5 mm.

*Bioaffinity* refers to the natural binding affinity of biomolecules between each other or a biomolecule to synthetically made specifically-binding reagents.

*Moving component* refers here to an individually moving component enabling to accomplish the desired analytical process.

*Transport unit* or *piston* refers to the main operational platform in the present disclosure which contains a place for a sample, a piston frame for protruding into reagent/liquid compartment(s), waste container and other possible accessories, exemplified by electrical connections.

*LOC body* refers to the outer part of the cassette which accommodates all the moving parts. In the case that the said LOC body is made of two parts, liquid reservoir and a space accommodating the piston in the preparedness stage, the latter is called as *chassis.* In the case LOC body is not divided into two parts, i.e. the liquid is included in specific plastic bags, the whole LOC body is called chassis.

*Sample preparation unit* or *SPU* refers here to an independently moving component in LOC to which has an opening for a crude analytical sample to be applied. The unit is able to divide the sample to analytically interesting and non-interesting portions. The unit is the basis for this device to be capable of providing an exact predetermined volume of a sample for a reaction. SPU can also provide chemicals like buffers, solubilizers, and/or other reagents needed in steps of the analysis process. SPU can consist of more than one connected parts which can slide in the respect of each other.

*Filtration unit* may *be* part of SPU which can be brought over the sample cavity and removed from that place before the measurement stage.

*Sample cavity* is the place into which the standardized and optionally purified sample is introduced. It can be a plane with bioactive surface, or a surface in a hollow, or cavity with similar, or related functions as the plane.

*Liquid reservoir* refers here to a container which is filled with a liquid needed for washing the sample before analysis or for performing the analysis steps after applying sample onto the sample cavity. In some embodiments the reservoir may also be empty. The liquid reservoir may be a detachable unit which is covered with a seal or foil. The container can be fitted to the main body of LOC device before its use. The fitting may be by snapping, screwing, sliding or any other feasible way. There can be more than one successive reservoir to be attached to the chassis with different solutions. In one version, the whole cassette body itself includes the liquid container(s) while the liquids are placed in that space in breakable plastic bag(s). Liquid reservoir is usually completely or partially transparent for visible light. It can also include a special window transparent to UV light or NIR.

*Waste reservoir* refers to a space whereto the surplus of used liquid(s) and reagents can flow. In a preferred construction the waste liquids flow to a cavity inside the piston.

### Overall construction and function

Figure 1 illustrates a device with the basic principles of the present disclosure. The construction depicts at least three mechanically moving parts or slides. The device comprises a chassis optionally consisting liquid reservoir(s) (4; in Fig 1) attached to one end of the chassis. The chassis may have a rectangular or a cylindrical form. A transport unit 3 having a sample cavity moves inside the chassis and can penetrate into liquid reservoir through a breakable seal and force liquid to move over the sample cavity 6 into waste reservoir 9 with the principle of displacement pump. Equally well, the liquid(s) can have been placed in separate plastic bags inside the chassis.

The sample preparation unit or SPU (2, in Figs 1) is formed of one or two sliding parts and is capable of sliding over the piston in direction of the longitudinal axis of the piston. Detailed construction of SPU depends on the analytical needs in concern. Figs 2 and 3 shows more complicated SPUs which can perform one or more functions exemplified by standardization of sample volume, dilution, addition of reagents and filtration.

Figure 2 (A-D) illustrates the simplest steps needed to take a volumetric sample into an analysis. The movements are coordinated so that each of the sequential steps has a meaningful function in performing the assay. After obtaining an accurate sample volume into the further analysis, various steps can be carried out and reagents can be added and the product can be purified. After incubation, if needed, the piston (3, in Fig 2) is pushed in to rinse the sample cavity (6, in Fig 2) and/or add other reagents. This construction allows large flexibility to generate various analytical concepts.

The assembly of Fig. 1 and the involved components form a cassette which functions as a LOC device. The sample preparation unit (SPU) is accommodated to fit into chassis together with the transport unit. Jointly they form a union which can move together independently of each other in back and forth in the direction of the longitudinal axis of the chassis. A sample is applied into a measuring opening in the SPU directly or through a hole in chassis (shown in Figs 1-5). The transport unit contains sample cavity which can serve as a reaction place for the treated and/or purified sample. It can be a special surface in a plain or in a depression in the material of the transport unit. A reservoir for the waste liquid (if any) is conveniently made inside the transport unit (as in Fig 1) or into a space under the piston. The piston frame of the transport unit contains sharp elements like spikes or sharp edges which break the seals closing the reagent reservoir(s), or plastic bags containing reagents inside the reservoir. The reagent reservoir(s) can be conveniently separate attachable units which can be fitted to the main body by pressing, sliding. The advantage of such construction is that their manufacturing and filling are easy. The reagent reservoirs are usually made of transparent materials but if the detection is made by an electrochemical cell they do not need to be transparent like in photo-detection. The overall dimensions of the devices such as shown in Fig. 1 are not critical for the function of the device. The essential feature is only the relative dimensions of the moving components and their fitness to each other to make logical functions.

An accurate sample volume is applied into sample measuring opening 5 in the SPU 2 in device shown in Fig 1. Alternatively, a bigger but undefined sample volume is applied to a more complicated version in Fig 2A into the sample opening 5 of the upper slide 10 aligned with the measuring opening 7 in the lower slide 11 to provide standardization of the sample volume after the steps shown in Fig.2 (A-D). While SPU of Fig 2 exemplifies the standardization of the sample volume, the SPU can be modified to carry out also other functions like purification of the sample, mixing of dry or liquid ingredients stored in the sample preparation unit in dry or liquid stage. From SPU, the liquid sample is driven either passively or with the aid of mechanical forces onto the place of reaction, e.g., sample cavity, wherein the next reaction(s) can be carried out. SPU may be transferred in the respect of transport unit at this step along the longitudinal axis of the chassis forth or back.

While the SPUs of Fig 2 have been described as being individual plates moving along the longitudinal axis of the chassis, it is equally well possible to divide axially the slides (e.g. 10 and 11 in Fig 2) into two or more rectangular strips which slide parallel and individually. This allows construction of devices to provide two or more samples in different volumes, for example, for multi-tests. The sample cavity is correspondingly divided into two or more compartments. This is feasible to arrange in case of e.g. electrochemiluminescence methods so that each compartment is excited separately. It is also possible to make non-rectangular shapes of the slides of Fig 2 (10) and/or (11) and provide very complicated two or even three dimensional movements and, correspondingly, to provide complex reaction conditions and operations with the same simple principles described in Figs 1-4 and elsewhere herein. Figure 5 illustrates a feasible construction allowing dispensing two equal or different samples into the same cassette.

The sample cavity (4; Fig 1-5)) may contain other specific reagents as prepared beforehand. After an appropriate incubation time with the sample, transport unit is moved towards the liquid reservoir(s) attached to the main body. The edges of the head of the transport unit break the seal of liquid reservoir(s) which contain(s) washing buffers or additional reagents or both. The head of the transport units will function as a replacement pump and this effects that the liquid in reservoir(s) move(s) to the sample cavity in a way what is meaningful for the operation. The first liquid container may have washing liquid for the sample cavity which removes unwanted or interfering materials and reagents from the sample cavity to waste reservoir through suitable holes. A second reservoir can contain further reagents which increase the specificity of the employed reagents. The second reagent is exemplified by a substrate of alkaline phosphatase which will be subject of hydrolysis and enzyme- labelled antibody producing a colour which can be measured. There can be also other successive reservoirs which contain other reagents exemplified with sodium carbonate solution stopping enzyme reaction of alkaline phosphatase and reinforcing the yellow colour. The volumes and concentrations of the reagents can be adjusted according to the sizes of the reaction chambers and needs of the liquids for the measurement purposes. For example, the substrate solution of alkaline phosphatase remains in the final position of the piston (liquid reservoir) and is closed to it with the aid of the brims (shown in Figs 1-2) of SPU. The waste reservoir is adapted to accommodate the waste liquid generated in different stages of the reaction steps. When the transport unit is at the final position, SPU is totally inside and SPU and the transport unit tighten the cassette so that there is no leakage to outside. Technically this is accomplished with correct dimensions of the moving parts with consideration of the features of the employed materials.

### Sample preparation unit (SPU)

Biochemical analyses usually start with a sample preparation that may be complicated and critical to the reliability of the results. The demanding samples are exemplified by body fluids, blood, urine, spine liquor, saliva, and so on, containing a complex mixture of cells, cell debris, salts, proteins, sugars, including the specific component of interest.

Environmental and industrial samples require removal of various solid materials (microbes, fibres etc.) from the samples. Purification is of extreme importance not only if any micro-channels or micro-machines are employed, but also because of possible interference to the analysis. The sample preparation unit SPU is preferably made of plastic, like polyethylene, polypropylene, polystyrene, or PVC, or PTFE. It can also contain more than one material including metals or covered with a metal vapour. A commonly used sample preparation system is filtration separating the desired fraction of particles and molecules allowing analytes to move forward but immobilizing interfering substances. There are two principally different filters, membrane filters and in-depth filters. Either one form can be exploited in the present disclosure. Filters allow small molecules to pass but make retention to big particles. Dialysis is also possible instead of filtration. In that case filtration membrane is substituted with a dialysis membrane and sample cavity is filled with a liquid. According to the present disclosure, it is also possible to concentrate big molecules and slow or prevent the movement of small molecules. This can be done with various gels used for size-exclusion chromatography as applied in dry or wet stage. It is also possible to use both filters and the said gels together to get increased concentration of the big-molecular fraction. The gels can be thereby confined inside two membrane layers bound together with a frame.

The objective of the present disclosure was to overcome drawbacks and complications in the prior art in the sample preparation and construct a robust LOC device which can be used outside of research laboratories. The standardization of small sample volumes is difficult to unexperienced persons and even to professionals outside special laboratory facilities.

SPU of the present disclosure forms a significant improvement over the prior art of the LOC devices to be used in POC conditions. SPUs can have different functions depending on the exact needs of the analysis.

A critical step, and often the most important in all quantitative analyses, is the quantifying the volume/amount of the sample volume. It was surprisingly found that simple movements which are related and can be logically combined to the other mechanical movements of the device will provide means to quantify or standardize the sample volume. That is, quantification can be achieved with the same principle as the other functions of the present LOC device, by macroscopic mechanical movements as exemplified in Fig 2. A core component is a slide which is constructed to have a hole with preadjusted volume. This is accompanied with another slide able to exclude indeterminate part from a bigger sample volume. The indeterminate volume is transferred to a waste storage (Fig.2B-C, 9). The standardized sample volume is applied to further processes by bringing an opening to allow the sample to be moved forward. The proper function of the slides of Fig 2 can be achieved in practise either by organizing O-ring seals around the sample holes or by using hydrophobic materials like siliconization around and/or inside the holes so that the samples will not move with capillary forces between the slides and come out from the hole when the hole is open from both upper and lower ends.

Often, especially in biological analyses, the sample must be diluted to overcome too high viscosity of the sample to dilute interfering substances below a significant level, or to fit the results into a suitable linear measuring range, and so on. SPUs of Fig 2 suit also for making those functions. The simplest way is that the hole or opening in the slide above the sample slide is filled with a dilution liquid which rinses the sample forward into the analysis procedure. The same stage can also involve reactive compounds needed for the analysis. Moreover, more than two slides with reagents can be included in SPU to enable it to make very sophisticated functions and reactions.

A dual measurement system with a single cassette may also be constructed with the principles of this invention. The sample cavity was divided into two parts having different functions. Except with two separate set of holes in slides 10 and 11, the problem can be solved conveniently as schematically shown in Fig. 5. Slide 11 will have two parallel holes for obtaining exact sample as 7 in Fig. 2. Moreover, a rack wheel or clog is mounted on 2 with a shaft. This rack wheel has holes corresponding to openings 5 in Fig 2 which holes are concentric with the pairwise measuring openings 7 on 2. These openings may be of same or different sizes. The positions of the openings in the rack wheel is adjusted with a rack bar related to the slide 10 in Fig 2. If needed a separate gear rack wheel can be added to construction to make the positioning of the rack wheel more accurate. The construction of Fig.5 suits also for one sample. Even the construction of Fig 5 is slightly more complicated than Figs 1-4, its advantage is that several different steps in adding reagents and buffers etc. can be included easily and different exact volumes can be applied by selecting which of the holes in the rack wheel are exploited.

Even though the main embodiment of the present disclosure is to exploit mechanical macroscopic movements of sliding parts, the overall construction allows easy inclusion of other functions controlled by electricity and devices even in micro-meter dimensions. However, herein the micro-functions are considered as additional sub-functions of the general principles of the present disclosure. The sub-functions can be achieved with micropumps, actuators, sensors, and so on. The polymerase chain reaction can be also carried out by using electrical heating and cooling systems, such as Peltier elements.

*Dry chemistry* is conveniently provided in the SPU stage by adding reagents needed to the analysis. Dried chemicals, in quantified amounts can have been dried in different locations in SPU. For example, for a sandwich bioaffinity assay, a detecting antibody could have been dried on the walls of the sample opening of SPU from where the sample, or a diluted sample, makes it available for a bioaffinity reaction in the sample cavity. The dried reagents for a polymerase reaction can be used as a mixture of dried primer, nucleotide probe, with other needed ingredients. The dried reagents can be provided in any other step of the reaction equally well, for example during the filtering process.

*Filtration as a part of SPU* is preferably applied as a step which excludes interfering materials from the sample to be analysed. The principle of that step is to allow the analytically important molecules to enter the stage of the next reaction step. Often, biological samples must be pre-treated by filtration for separating cells, cell debris, aggregated proteins etc. Therefore, the LOC device contains a membrane or in-depth filter. They can be fitted, into a special frame or can be rolled off when the piston is moved. Essential feature is that the frame for the filter is slightly lifted during movement of the transport unit forward (as implied in Fig 3). This prevents the membrane spoiling the bioactive surface and at the same time it locks the cassette from leaking. These and other traits make a significant improvement over US 9,463,457 B2 (Conventa Ltd.) wherein an optional membrane was removed by the injection of the fluid from the liquid reservoir. For the multitude of unique details in SPU, the present disclosure can be called as the LOC device and not only as washing and measuring instrument.

### Transport unit

The transport unit, or more descriptively piston is the main platform of all of the functions of the current LOC device. It can be constructed in various ways with different forms and materials. Preferred constructions are shown in Figs 1-4. The exact construction depends largely on the analysis type to be carried out. The transport unit can also include the waste reservoir to which waste liquid is driven through holes over the sample cavity. The transport unit can contain functions like electric wiring and connections (Fig.4).

### Sample cavity

The sample cavity, can have different functions. It can have only a plain bioactive surface, a hollow or cavity, or a more complicated structure like in an electrochemical cell. I.e. it can have different forms and properties depending on the type of the analytical procedure. The plain surface can be a polystyrene surface, commonly used in microtiter plates for making the sandwich bioaffinity assays. The surface can be also modified to increase its surface area with pillars, holes, grooves, or it may have a hollow accommodating liquids, dried reagents etc. The outer surface of the sample cavity can be also brought from outside and fixed or glued onto the place of the sample cavity. There can be also more than one type of surfaces modified in different ways, e.g. coated with different antibodies, oligonucleotides, or other specific probes. The surface can include dried chemically reactive functions, exemplified with anhydrides or oxirane groups which will be exploited further. The surface material can be polystyrene film, an electronic unit, like a FET transistor, silicon, or a metal or conductor chip for ECL detection, or an electrochemical cell. Especially the electrochemical surfaces can be obtained with micro- or nanotechnological means to have conductor spikes with polymers or their metal polymer composites. The electrochemical surfaces can be also manufactured at the sample cavity with ways exemplified by etching, spraying, sputtering, spin coating, painting, or the surface is added in the form of adhesive tape. The surface can be coated with magnetic particles which are fixed on their place by a magnetic field during the washing and transferred into the stage of detection (liquid reservoir) wherein the magnet may be released allowing the particles to distribute into the whole liquid measuring volume. The sample cavity may be covered with a membrane or filter during the required chemical and/or biochemical reactions and detection.

The sample cavity is often a polystyrene surface which is very common material of microtiter plates. The transport unit can be any polymer, like polystyrene, and hence bioactive surface can be a part of the transport unit. Sample cavity can have a well of depth of 0.1 - 5 mm which may have been coated directly with catching antibodies or with nucleotide probes passively or covalently or through intermediators like, covalently reacting chemicals, or streptavidin mediators. The sample cavity may have been also covered with other materials than the material of the transport unit, exemplified by silicon, a metal, silicon oxide, carbon paste or a plastic composite containing carbon paste or another conductor than carbon paste. The surfaces may have been covered with a filter material.

### Waste reservoir

The purpose of the waste reservoir is to manage with reagent or microbial wastes inside the cassette and not allowing them to make even a potential health risk to the users. The reservoir can be organized in various ways inside the cassette. A convenient way is inside the piston as in Fig 1 (9). The income of the liquid can take place through holes above or aside the reaction cavity so that it will be rinsed with required solvents. The air can be introduced out from the cassette through holes which are closed at the very end of the movements (closing stage of the cassette). The waste reservoir can be also organized outside the piston part. The reservoir can contain a material stabilizing the waste liquid into a form of a gel and preventing its leakage and thereby further ensuring tightness of the system from leakages.

### Liquid reservoir(s)

Majority of analyses are made in solutions and therefore the present disclosure contains one or more of liquid containers. In a preferred mode the reservoir is filled separately with a liquid needed in the assay and stored while connected to the other units of the cassette as indicated in Figs 1-4. When the liquid reservoir is made separately, it is usually closed air-tightly with a breakable seal, like plastic or plastic and aluminium. The material of the liquid reservoir is chosen so that it does not dissolve or corrode by its content and allows measurement of the analysis results.

However, the liquid reservoir(s) can be also used differently according to the present disclosure. The reservoir(s) can contain gases other than air, such as nitrogen, carbon dioxide, nitrogen oxides, or mixtures of gases and liquids, or materials impregnated with evaporating or sublimating substances, solids which react with the evolving gases. Such LOC devices are exemplified by cultivation of microbes, especially for collecting hazardous microbiological samples. The reservoir can in such case form a locked incubation chamber while and after a certain time the amount of the microbial colonies on a growth medium in sample cavity is calculated through a window. This can be done visually or by an automatic image analyser system or by developed colour or other known methods used for microbiological identification and quantitation.

*Washing step* of the sample cavity is carried out usually after the specific reactions. Bioaffinity reaction may have allowed to proceed to its completion or stopped after exact timing. The piston is being transferred to its final position while the washing liquid flows over the sample cavity and then enters the waste container. This step can include also addition of other reagents. In some applications of the device according to the present disclosure, additional reagents are added to detect the analyte, exemplified by an ELISA assay. The speed of the washing step can be controlled by an external device.

*Measurement,* or detection can be done simplest visually by comparing with colour standards. More often, however, recorded quantitative results are required. This can be done with various ways depending on the analysis to be carried. The physical method is exemplified by electronic absorption, turbidity, fluorescence, fluorescence polarization, luminescence, or amperometry.

### Alternative applications of LOC devices

If the liquid reservoir(s) do(es) not contain liquid(s), the function of the LOC device is slightly different while the construction can be the same. Example 2 illustrates how bacterial forms are separated from a sample and introduced into a sample cavity on the transport unit the sample cavity being filed with specific solid growth medium. Medium can be washed and moistened with a pulse of growth liquid from the liquid reservoir with a movement of the transport unit. After this, the microbial sample is transferred onto to the sample cavity and the allowed to infect the medium. Thereafter the transport unit is moved forward to the incubation position with the sample cavity locating close to the distal end of the liquid reservoir. The incubation can be conducted in desired conditions for longer periods in microbially safe conditions and inspected through a transparent window whenever needed. Anaerobic cultivations can be made obtaining the sample cavity with microbial inoculation to penetrate into liquid reservoir filled with solid or semisolid growth agar.

Example 4 and Figure 4 illustrate a construction wherein the present LOC device is connected to a source of electricity which source includes measuring instrument. A preferred application of the present disclosure is to use the LOC in connection with electrical excitation of fluorescent labels in bioaffinity assays. In such applications the measurement instrument provides electrical pulses to the LOC device, which pulses excite the label molecules and the generated fluorescence and/ or luminescence is recorded and analysed. Such luminescence methods deviate from many other possible applications of the LOC described here only by the existence of electrical connections to the sample cavity.

### Variations of the construction and functions

The LOC device according to the present disclosure can be used independently of any additional instrument. After sample application, the required mechanical processes are conducted manually and the results are estimated visually. Alternatively, after the application of the sample, the LOC device is connected to an instrument which will make the mechanical movements and measure the results. The latter version of the instrument can be more sophisticated since various other functions can be readily included in the LOC device. For example, it is possible to get electricity from the measuring instrument to carry out other processes than the movements, that is, to warm and/or cool the device, to get signals from actuators, measure parameters like temperature, humidity, position of the mechanical step, run other processes like pumps, mixers, centrifuges etc.

The scope in the view of the present disclosure was to develop a simple device so that unprofessional persons can conduct sophisticated analyses from biological samples. This can be a related issue to revolution in the sphere of personal computers and internet which were considered belonging only to limited group of professionals but are now part of everyday life. The present disclosure provides a simple but versatile device involving a set of chemical tools of dry chemistry, sample preparation, washing, and chemical reactions followed by detection with a physical method or visually. If the LOC cassette is used with measuring device involving electricity, it is easy to include even very complex electrically operated and guided functions in combination with mechanical movements.

The LOC device of is illustrated below further with non-limiting Examples.

### EXAMPLE 1.

### Application of the LOC device to electrochemical analysis of CRP

A device depicted in Fig 4 was constructed. It had an oval cross sectional form which had an advantage over the rectangular form for minimizing possible liquid leakages. The length of the devise in the sealed position was about 6 cm and the other dimensions related to the length as in Fig. 1. The cassette was employed for measuring cathodic electrochemiluminescence essentially as in a system previously described by US 9,463,457 B2 (Conventa Ltd.). The oxidized silicon chip of 6x6 mm was embedded in the sample cavity and electrical wiring was arranged with the silver printing method on a plastic foil which was then attached onto the transport unit made of polycarbonate. Alternatively, the sample cavity was spin-coated with carbon powder and polystyrene dissolved in acetone and allowed to dry. The piston frame of the transport unit (3 in Fig 4) was equipped with sharp edges which break the seal of the reagent reservoir when pushed down into the solution and which liquid then washes the silicon chip.

A blood sample of 10-50 microliters was applied onto the sample hole depicted in more detail in Fig 2. The transport unit was then moved in such a way that excess of the blood sample was cut from slide (Fig 2, step 2) and transferred to idle place whereas the lower slide including exactly known sample volume of 3.5 microliter was transferred over the sample application place. When the hole of the upper slide was fitted on the exact sample side, the sample drops onto the filtration system bound to lower surface of SPU. Membrane also include dried Tb-chelate -labelled antibody against C-reactive protein (CRP). The filter removed the blood cells while allowing other blood components to pass and distribute onto the antibody -coated silicon surface. The C-reactive protein- antibody-label complex binds onto the surface to the capture antibodies coated onto the silicon and forms a sandwich antibody-antigen-labelled antibody complex. After a 2 -minute incubation, the transport unit is pushed down, SPU with the membrane is slightly lifted up by an elevation in the frame of the transport unit. The sharp edges at the frame of the transport unit break the seal of the liquid reservoir (1.7 mL) and the protruding washing liquid rinses the silicon surface while SPU with the membrane stops before the transport unit reaches the bottom of the liquid reservoir. The rinsing liquid is introduced into the waste reservoir through the holes in the transport unit. A liquid layer is left also in the sample cavity over the silicon chip. When the transport unit was pushed all the way in such that the sample cavity is close to the bottom of the liquid reservoir, electrical pulses of 10-30 V were applied between cathode and anode and the resulting luminescence was measured through the transparent wall of the liquid reservoir with a time-resolved mode. The results were in accordance with the previous results without applying the cassette and the standardization of the sample volume.

### EXAMPLE 2

### Application of the LOC device to measuring viable bacteria in waste water

The sample cavity of LOC device of Fig. 3 was filled with liquid nutrient agar with size of 1x6x6 mm. Water to be analysed was added aseptically through the sampling unit (accurate sample volume 3.5 microL) was taken through the sampling system. The second hole of the SPU contained 50 microL of 0.9% NaCl solution. When the slides were transferred over the agar layer, the sample was rinsed with 50 microL the salt solution over the agar and distributed on it. The piston was moved down toward the empty liquid reservoir while the slides (10) and (11) were lifted slightly up not to touch the agar. The piston was moved totally to the end position. The closed LOC device was then transferred to a place of 37° C and inspected for viable colonies on the next day.

### EXAMPLE 3

C-reactive protein (CRP) was measured from human whole blood essentially as in US 9,463,457 B2 (Conventa Ltd.) with using an antibody linked to alkaline phosphatase enzyme with a device as shown in Fig.4. The liquid reservoir contained 1.3 mL of the substrate solution of alkaline phosphatase in the first compartment of the liquid reservoir and 0.4 mL of sodium carbonate solution in the next compartment. The surface of the sample cavity was coated with CRP primary antibody while the labelled secondary antibody was dried on the membrane. After the immune reaction (2 min) the transport unit was changed to so that the first membrane of the liquid reservoir was broken and the extra alkaline phosphate solution was flowing to the waste reservoir. After 30 min incubation at the room temperature the second membrane wall was disrupted and the intensity of the yellow colour was measured.

## Claims

1. A lab on a chip device to accurately measure an exact volume of a sample and conduct a chemical or biochemical assay, **characterized in that** said device comprises :
a hollow chassis (1) attachable to a liquid reservoir (4) and comprising a sample application hole, a sample preparation unit (SPU) (2) comprising at least one slide (10, 11) having a sample application opening (5) extending through the slide (10) and having a defined volume, and a transport unit (3) comprising a sample cavity (6) comprising reagents, wherein the SPU (2) and the transport unit slide in relation to each other and inside the chassis (1), and the sliding movement allows alignment of the sample application hole in the chassis (1) and the sample application opening (5) in the SPU (2), thus enabling filling the sample application opening (5) with a sample, and wherein further sliding movement allows alignment of the sample application opening (5) in the SPU (2) and the sample cavity (6) in the transport unit (3), thus enabling filling the sample cavity (6) with a standardized volume defined by the volume of the sample application opening (5), and
wherein the sample cavity (6) can be moved by sliding of the transport unit (3) into the liquid reservoir (4) for further reactions or washing, and
wherein said SPU (2) comprises two slides (10, 11):
an upper slide (10) having at least one sample application opening (5) and an air hole (8) both extending through the upper slide (10); and
a lower slide (11) having at least one sample measuring opening (7) extending through the lower slide (11) and having a predetermined exact volume;
the device having a sample application position, a sample standardization position, a sample transfer position, and a sample washing position, wherein
in the sample application position the upper slide (10) and the lower slide (11) of the SPU (2) are positioned inside the chassis (1) such that the sample application hole of the chassis (1), at least one sample application opening (5) of the upper slide (10) and at least one sample measuring cavity (6) of the lower slide (11) are aligned with each other, thus allowing a sample to enter through the sample application hole and fill the application opening (5) and the sample measuring opening (7);
in the sample standardization position the upper slide (10) being positioned such that the at least one sample application opening (5) of the upper slide (10) and the at least one sample measuring opening (7) of the lower slide (11) are misaligned, and contents of the at least one sample application opening (5) are thus contained in the opening while the at least one sample measuring opening (7) contains the exact predetermined volume of the sample;
in the sample transfer position the upper slide (10) and the lower slide (11) of the SPU (2) and the transport unit (3) are positioned inside the chassis (1) such that the sample application hole of the chassis (1), the air hole (8) of the upper slide (10), the at least one sample measuring opening (7) of the lower slide (11) and the sample cavity (6) in the transport unit (3) are aligned with each other, thereby allowing the exact predetermined volume in the at least one measuring opening (7) to enter the sample cavity (6) and allowing an option to add subsequent reagents to the sample cavity (6) through the aligned openings;
in the sample washing position the SPU (2) is positioned completely inside the chassis (1) and the sample transfer unit is positioned such that the sample cavity (6) locates in the liquid reservoir (4).

2. The device of claim 1, wherein the upper slide (10) has a toothed rack bar and the lower slide (11) is a rotating rack wheel, wherein the rack wheel has a multitude of sample measuring openings (7) of same or different exact volumes, thereby allowing exact measurement of more than one sample or reagent onto the sample cavity (6).

3. A lab on a chip device according to claim 1 wherein
the hollow chassis (1) has a first open end, and a second open end;
the detachable liquid reservoir (4) has a bottom, at least partially transparent closed wall and an upper open end optionally covered with a membrane, said reservoir (4) containing reagents necessary for the assay, and said reservoir (4) being connectable from the upper end to the first open end of the chassis (1);
the transport unit (3) being a piston having a longitudinal axis and moving into the chassis (1) from its second open end, said transport unit (3) having a piston frame and a sample cavity (6) optionally covered by a filtering unit and containing predetermined reagents; and
the sample preparation unit (SPU) (2) locating on top of the transport unit (3) and being capable of sliding along the longitudinal axis of the piston and the SPU (2) having atl least the function of standardization of sample volume.

4. The device according to claim 3, wherein the upper end of the liquid reservoir (4) is covered with a membrane and the distal end of the piston frame has sharp elements capable of breaking the membrane when the piston in pushed into its position in the washing position.

5. The device according to claim 3 or 4, wherein the piston frame has a waste liquid reservoir (9).

6. The device of any one of the previous claims, wherein the sample cavity (6) contains an electric chip and electrical connections locate on the end plate of the piston.

7. The device of any one of the previous claims, wherein the sample cavity (6) is covered with a material different from material of the transport unit (3), said covering material being selected from polystyrenes, plastics, silicon, aluminium, a metal covered with thin flawless resistor layer, carbon paste, a composite material consisting of a conductor and a polymer, and wherein the sample cavity (6) is optionally further covered with a bioactive surface enabling bioaffinity reaction with the sample and said cavity (6) locating under a filter unit attached to the sample preparation unit.

8. A method to accurately measure an exact volume of a sample and conduct a chemical or biochemical assay, said method comprising the steps of:
a. providing a device according to any one of claims 1-7;
b. applying a sample in excess to needed volume on the sample hole when the device is in a sample application position,
c. cutting the sample into two parts by transferring the device into a sample standardization position, where one part of the sample is in the sample application opening (5) moved in an idle position and the other part is in the sample measuring opening (7) and having the exact volume required for the reaction;
d. allowing the exact volume of the sample to drop to a sample cavity (6) optionally through a filtering unit, said sample cavity (6) containing reagents suitable for the required reaction by transferring the device into a sample transfer position;
e. allowing the sample to react with the reagents in the sample cavity (6) for a required period and thereafter transferring the device into washing position by moving the sample transfer unit (3) such that the sample cavity (6) is in the liquid reservoir (4); and
f. measuring a chemical or biochemical reaction through the transparent wall of the liquid reservoir (4) by recording a visual, fluorometric, colorimetric, spectrophotometric or luminometric change.

9. The method of claim 8, wherein the sample cavity (6) contains an electric chip and electric connections from the chip locate on the end plate of the piston, wherein the reaction in the sample cavity (6) is an electrochemiluminescence reaction.

## Patentansprüche

1. Labor-auf-Chip-Vorrichtung, um ein genaues Volumen einer Probe genau zu messen und einen chemischen oder biochemischen Assay durchzuführen, **dadurch gekennzeichnet, dass** die Vorrichtung Folgendes umfasst:
ein hohles Chassis (1), das an einem Flüssigkeitsreservoir (4) angebracht werden kann und ein Probenauftragslochs umfasst, eine Probenvorbereitungseinheit (SPU) (2), umfassend mindestens einen Objektträger (10, 11) mit einer Probenauftragsöffnung (5), die sich durch den Objektträger (10) erstreckt und ein definiertes Volumen aufweist und eine Transporteinheit (3), umfassend einen Probenhohlraum (6), umfassend Reagenzien, wobei die SPU (2) und die Transporteinheit relativ zueinander und innerhalb des Chassis (1) gleiten, und die Gleitbewegung die Ausrichtung des Probenauftragslochs im Chassis (1) und der Probenauftragsöffnung (5) in der SPU (2) ermöglicht, wodurch das Füllen der Probenauftragsöffnung (5) mit einer Probe ermöglicht wird, und wobei eine weitere Gleitbewegung die Ausrichtung der Probenauftragsöffnung (5) in der SPU (2) und des Probenhohlraums (6) in der Transporteinheit (3) ermöglicht, wodurch das Füllen des Probenhohlraums (6) mit einem standardisierten Volumen ermöglicht wird, das durch das Volumen der Probenauftragsöffnung (5) definiert ist, und
wobei der Probenhohlraum (6) durch Gleiten der Transporteinheit (3) in das Flüssigkeitsreservoir (4) für weitere Reaktionen oder Waschen bewegt werden kann, und
wobei die SPU (2) zwei Objektträger (10, 11) umfasst:
einen oberen Objektträger (10) mit mindestens einer Probenauftragsöffnung (5) und einem Luftloch (8), die sich beide durch den oberen Objektträger (10) erstrecken; und
einen unteren Objektträger (11) mit mindestens einer Probenauftragsöffnung (7), die sich durch den unteren Objektträger (11) erstreckt und ein vorbestimmtes genaues Volumen aufweist;
wobei die Vorrichtung eine Probenauftragsposition, eine Probenstandardisierungsposition, eine Probentransferposition und eine Probenwaschposition hat, wobei in der Probenauftragsposition der obere Objektträger (10) und der untere Objektträger (11) der SPU (2) innerhalb des Chassis (1) positioniert sind, so dass das Probenauftragsloch des Chassis (1), mindestens eine Probenauftragsöffnung (5) des oberen Objektträgers (10) und mindestens ein Probenmesshohlraum (6) des unteren Objektträgers (11) miteinander ausgerichtet sind, wodurch eine Probe durch das Probenauftragsloch eintreten und die Auftragsöffnung (5) und Probenmessöffnung (7) füllen kann;
wobei in der Probenstandardisierungsposition der obere Objektträger (10) so positioniert wird, dass die mindestens eine Probenauftragsöffnung (5) des oberen Objektträgers (10) und die mindestens eine Probenmessöffnung (7) des unteren Objektträgers (11) falsch ausgerichtet sind, und der Inhalt der mindestens einen Probenauftragsöffnung (5) somit in der Öffnung enthalten ist, während die mindestens eine Probenmessöffnung (7) das genau vorbestimmte Volumen der Probe enthält;
wobei sich der obere Objektträger (10) und der untere Objektträger (11) der SPU (2) und der Transporteinheit (3) in der Probentransferposition im Chassis (1) befinden, so dass das Probenauftragsloch des Chassis (1), das Luftloch (8) des oberen Objektträgers (10), die mindestens eine Probenmessöffnung (7) des unteren Objektträgers (11) und der Probenhohlraum (6)) in der Transporteinheit (3) aufeinander ausgerichtet sind, wodurch das genau vorbestimmte Volumen in der mindestens einen Messöffnung (7) in den Probenhohlraum (6) eintreten kann und eine Option zum Hinzufügen nachfolgender Reagenzien zum Probenhohlraum (6) durch die ausgerichteten Öffnungen ermöglicht wird;
wobei sich die SPU (2) in der Probenwaschposition vollständig im Chassis (1) befindet und die Probentransfereinheit so positioniert ist, dass sich der Probenhohlraum (6) im Flüssigkeitsreservoir (4) befindet.

2. Vorrichtung nach Anspruch 1, wobei der obere Objektträger (10) eine gezahnte Zahnstangenstange aufweist und der untere Objektträger (11) ein rotierendes Zahnstangenrad ist, wobei das Zahnstangenrad eine Vielzahl von Probenmessöffnungen (7) mit gleichen oder unterschiedlichen exakten Volumina aufweist, wodurch eine exakte Messung von mehr als einer Probe oder einem Reagenz auf dem Probenhohlraum (6) ermöglicht wird.

3. Labor-auf-Chip-Vorrichtung nach Anspruch 1, wobei das hohle Chassis (1) ein erstes offenes Ende und ein zweites offenes Ende aufweist;
das abnehmbare Flüssigkeitsreservoir (4) eine untere, zumindest teilweise transparente geschlossene Wand und ein oberes offenes Ende aufweist, das gegebenenfalls mit einer Membran bedeckt ist, wobei das Reservoir (4) Reagenzien enthält, die für den Assay erforderlich sind, und das Reservoir (4) vom oberen Ende mit dem ersten offenen Ende des Chassis (1) verbindbar ist;
wobei die Transporteinheit (3) ein Kolben mit einer Längsachse ist und sich von seinem zweiten offenen Ende in das Chassis (1) bewegt, wobei die Transporteinheit (3) einen Kolbenrahmen und einen Probenhohlraum (6) aufweist, die gegebenenfalls von einer Filtereinheit bedeckt sind und vorbestimmte Reagenzien enthalten; und wobei sich die Probenvorbereitungseinheit (SPU) (2) oben auf der Transporteinheit (3) befindet und in der Lage ist, entlang der Längsachse des Kolbens zu gleiten, und die SPU (2) mindestens die Funktion der Standardisierung des Probenvolumens hat.

4. Vorrichtung nach Anspruch 3, wobei das obere Ende des Flüssigkeitsreservoirs (4) mit einer Membran bedeckt ist und das distale Ende des Kolbenrahmens scharfe Elemente aufweist, die die Membran brechen können, wenn der Kolben in seine Position in der Waschposition gedrückt wird.

5. Vorrichtung nach Anspruch 3 oder 4, wobei der Kolbenrahmen ein Abfallflüssigkeitsreservoir (9) aufweist.

6. Vorrichtung nach einem der vorhergehenden Ansprüche, wobei der Probenhohlraum (6) einen elektrischen Chip enthält und elektrische Verbindungen auf der Endplatte des Kolbens angeordnet sind.

7. Vorrichtung nach einem der vorhergehenden Ansprüche, wobei der Probenhohlraum (6) mit einem Material bedeckt ist, das sich von dem Material der Transporteinheit (3) unterscheidet, wobei das Abdeckmaterial ausgewählt ist aus Polystyrolen, Kunststoffen, Silizium, Aluminium, einem Metall, das mit einer dünnen einwandfreien Widerstandsschicht bedeckt ist, einer Kohlenstoffpaste, einem Verbundmaterial, das aus einem Leiter und einem Polymer besteht, und wobei der Probenhohlraum (6) gegebenenfalls ferner mit einer bioaktiven Oberfläche bedeckt ist, die eine Bioaffinitätsreaktion mit der Probe ermöglicht, und der Hohlraum (6) sich unter einer Filtereinheit befindet, die an der Probenvorbereitungseinheit angebracht ist.

8. Verfahren zum genauen Messen eines genauen Volumens einer Probe und Durchführen eines chemischen oder biochemischen Assays, wobei das Verfahren die folgenden Schritte umfasst:
a. Bereitstellen einer Vorrichtung gemäß einem der Ansprüche 1 bis 7:
b. Aufbringen einer Probe mit einem Überschuss auf das erforderliche Volumen auf das Probenloch, wenn sich die Vorrichtung in einer Probenauftragsposition befindet,
c. Schneiden der Probe in zwei Teile durch Überführen der Vorrichtung in eine Probenstandardisierungsposition, in der sich ein Teil der Probe in der Probenauftragsöffnung (5) in einer Leerlaufposition befindet, und sich der andere Teil in der Probenmessöffnung (7) befindet und das genaue Volumen hat, das für die Reaktion erforderlich ist;
d. Ermöglichen, dass das genaue Volumen der Probe optional durch eine Filtereinheit in einen Probenhohlraum (6) fällt, wobei der Probenhohlraum (6) Reagenzien enthält, die für die erforderliche Reaktion geeignet sind, indem die Vorrichtung in eine Probentransferposition überführt wird:
e. Ermöglichen, dass die Probe für einen erforderlichen Zeitraum mit den Reagenzien im Probenhohlraum (6) reagiert, und anschließend, Überführen der Vorrichtung in die Waschposition durch Bewegen der Probentransfereinheit (3), so dass sich der Probenhohlraum (6) im Flüssigkeitsreservoir befindet ( 4); und
f. Messen einer chemischen oder biochemischen Reaktion durch die transparente Wand des Flüssigkeitsreservoirs (4) durch Aufzeichnen einer visuellen, fluorometrischen, kolorimetrischen, spektrophotometrischen oder luminometrischen Änderung.

9. Verfahren nach Anspruch 8, wobei der Probenhohlraum (6) einen elektrischen Chip enthält und elektrische Verbindungen von dem Chip auf der Endplatte des Kolbens angeordnet sind, wobei die Reaktion in dem Probenhohlraum (6) eine Elektrochemilumineszenzreaktion ist.

## Revendications

1. Dispositif de laboratoire sur puce pour mesurer avec précision un volume exact d'un échantillon et réaliser un essai chimique ou biochimique, **caractérisé en ce que** ledit dispositif comprend :
un châssis creux (1) pouvant être fixé à un réservoir de liquide (4) et comprenant un orifice d'application d'échantillon, une unité de préparation d'échantillon (SPU) (2) comprenant au moins une pièce coulissante (10, 11) ayant une ouverture d'application d'échantillon (5) traversant la pièce coulissante (10) et ayant un volume défini, et une unité de transport (3) comprenant une cavité d'échantillon (6) comprenant des réactifs, dans lequel la SPU (2) et l'unité de transport coulissent l'une par rapport à l'autre et à l'intérieur du châssis (1), et le mouvement de coulissement permet l'alignement de l'orifice d'application d'échantillon dans le châssis (1) et de l'ouverture d'application d'échantillon (5) dans la SPU (2), permettant ainsi de remplir l'ouverture d'application d'échantillon (5) avec un échantillon, et
dans lequel un mouvement de coulissement supplémentaire permet l'alignement de l'ouverture d'application d'échantillon (5) dans la SPU (2) et de la cavité d'échantillon (6) dans l'unité de transport (3), permettant ainsi de remplir la cavité d'échantillon (6) avec un volume normalisé défini par le volume de l'ouverture d'application d'échantillon (5), et
dans lequel la cavité d'échantillon (6) peut être déplacée en faisant coulisser l'unité de transport (3) dans le réservoir de liquide (4) pour des réactions ou un lavage supplémentaires, et
dans lequel ladite SPU (2) comprend deux pièces coulissantes (10, 11) :
une pièce coulissante supérieure (10) ayant au moins une ouverture d'application d'échantillon (5) et un orifice d'air (8) traversant tous les deux la pièce coulissante supérieure (10) ; et
une pièce coulissante inférieure (11) ayant au moins une ouverture de mesure d'échantillon (7) traversant la pièce coulissante inférieure (11) et ayant un volume exact prédéterminé ;
le dispositif ayant une position d'application d'échantillon, une position de normalisation d'échantillon, une position de transfert d'échantillon et une position de lavage d'échantillon, dans lequel dans la position d'application d'échantillon, la pièce coulissante supérieure (10) et la pièce coulissante inférieure (11) de la SPU (2) sont positionnées à l'intérieur du châssis (1) de sorte que l'orifice d'application d'échantillon du châssis (1), au moins une ouverture d'application d'échantillon (5) de la pièce coulissante supérieure (10) et au moins une cavité de mesure d'échantillon (6) de la pièce coulissante inférieure (11) sont alignés les uns avec les autres, permettant ainsi à un échantillon d'entrer par l'orifice d'application d'échantillon et de remplir l'ouverture d'application (5) et l'ouverture de mesure d'échantillon (7) ;
dans la position de normalisation d'échantillon, la pièce coulissante supérieure (10) est positionnée de sorte que l'au moins une ouverture d'application d'échantillon (5) de la pièce coulissante supérieure (10) et l'au moins une ouverture de mesure d'échantillon (7) de la pièce coulissante inférieure (11) sont décalées, et le contenu de l'au moins une ouverture d'application d'échantillon (5) est ainsi contenu dans l'ouverture lorsque l'au moins une ouverture de mesure d'échantillon (7) contient le volume prédéterminé exact de l'échantillon ;
dans la position de transfert d'échantillon, la pièce coulissante supérieure (10) et la pièce coulissante inférieure (11) de la SPU (2) et l'unité de transport (3) sont positionnées à l'intérieur du châssis (1) de sorte que l'orifice d'application d'échantillon du châssis (1), l'orifice d'air (8) de la pièce coulissante supérieure (10), l'au moins une ouverture de mesure d'échantillon (7) de la pièce coulissante inférieure (11) et la cavité d'échantillon (6) dans l'unité de transport (3) sont alignés les uns avec les autres, permettant ainsi au volume prédéterminé exact dans l'au moins une ouverture de mesure (7) d'entrer dans la cavité d'échantillon (6) et permettant ainsi une possibilité d'ajouter des réactifs supplémentaires dans la cavité d'échantillon (6) à travers les ouvertures alignées ;
dans la position de lavage d'échantillon, la SPU (2) est positionnée complètement à l'intérieur du châssis (1) et l'unité de transfert d'échantillon est positionnée de sorte que la cavité d'échantillon (6) se situe dans le réservoir de liquide (4).

2. Dispositif selon la revendication 1, dans lequel la pièce coulissante supérieure (10) a une barre de crémaillère et la pièce coulissante inférieure (11) est une roue à crémaillère rotative, dans lequel la roue à crémaillère a une multitude d'ouvertures de mesure d'échantillon (7) de volumes exacts identiques ou différents, permettant ainsi une mesure exacte de plus d'un échantillon ou réactif sur la cavité d'échantillon (6) .

3. Dispositif de laboratoire sur puce selon la revendication 1, dans lequel
le châssis creux (1) a une première extrémité ouverte et une seconde extrémité ouverte ;
le réservoir de liquide (4) détachable a un fond, une paroi fermée au moins partiellement transparente et une extrémité supérieure ouverte éventuellement recouverte d'une membrane, ledit réservoir (4) contenant des réactifs nécessaires à l'essai, et ledit réservoir (4) pouvant être relié de l'extrémité supérieure à la première extrémité ouverte du châssis (1) ;
l'unité de transport (3) étant un piston ayant un axe longitudinal et se déplaçant dans le châssis (1) à partir de sa seconde extrémité ouverte, ladite unité de transport (3) ayant un cadre de piston et une cavité d'échantillon (6) éventuellement recouverte d'une unité de filtrage et contenant des réactifs prédéterminés ; et l'unité de préparation d'échantillon (SPU) (2) étant située au-dessus de l'unité de transport (3) et pouvant coulisser le long de l'axe longitudinal du piston et la SPU (2) ayant au moins la fonction de normalisation de volume d'échantillon.

4. Dispositif selon la revendication 3, dans lequel l'extrémité supérieure du réservoir de liquide (4) est recouverte d'une membrane et l'extrémité distale du cadre de piston a des éléments coupants pouvant rompre la membrane lorsque le piston est poussé dans sa position de lavage.

5. Dispositif selon la revendication 3 ou 4, dans lequel le cadre de piston possède un réservoir de déchets liquides (9).

6. Dispositif selon l'une quelconque des revendications précédentes, dans lequel la cavité d'échantillon (6) contient une puce électrique et des connexions électriques situées sur la plaque d'extrémité du piston.

7. Dispositif selon l'une quelconque des revendications précédentes, dans lequel la cavité d'échantillon (6) est recouverte d'un matériau différent du matériau de l'unité de transport (3), ledit matériau de couverture étant choisi parmi les polystyrènes, les plastiques, le silicium, l'aluminium, un métal recouvert d'une fine couche de résistance sans défaut, une pâte de carbone, un matériau composite constitué d'un conducteur et d'un polymère, et dans lequel la cavité d'échantillon (6) est éventuellement recouverte en outre d'une surface bioactive permettant une réaction de bioaffinité avec l'échantillon et ladite cavité (6) est située sous une unité de filtre fixée à l'unité de préparation d'échantillon.

8. Procédé pour mesurer avec précision un volume exact d'un échantillon et réaliser un essai chimique ou biochimique, ledit procédé comprenant les étapes suivantes :
a. la fourniture d'un dispositif selon l'une quelconque des revendications 1 à 7 ;
b. l'application d'un échantillon en excès par rapport au volume nécessaire sur l'orifice d'échantillon lorsque le dispositif est dans une position d'application d'échantillon,
c. la découpe de l'échantillon en deux parties en transférant le dispositif dans une position de normalisation d'échantillon, où une partie de l'échantillon est dans l'ouverture d'application d'échantillon (5) déplacée dans une position de repos et l'autre partie est dans l'ouverture de mesure d'échantillon (7) et ayant le volume exact requis pour la réaction ;
d. le fait de permettre au volume exact de l'échantillon de tomber dans une cavité d'échantillon (6) éventuellement à travers une unité de filtrage, ladite cavité d'échantillon (6) contenant des réactifs adaptés à la réaction requise en transférant le dispositif dans une position de transfert d'échantillon ;
e. le fait de permettre à l'échantillon de réagir avec les réactifs dans la cavité d'échantillon (6) pendant une période requise puis de transférer le dispositif dans la position de lavage en déplaçant l'unité de transfert d'échantillon (3) de sorte que la cavité d'échantillon (6) se trouve dans le réservoir de liquide (4) ; et
f. la mesure d'une réaction chimique ou biochimique à travers la paroi transparente du réservoir de liquide (4) en enregistrant un changement visuel, fluorométrique, colorimétrique, spectrophotométrique ou luminométrique.

9. Procédé selon la revendication 8, dans lequel la cavité d'échantillon (6) contient une puce électrique et des connexions électriques provenant de la puce situées sur la plaque d'extrémité du piston, dans lequel la réaction dans la cavité d'échantillon (6) est une réaction d'électrochimiluminescence.
